# EUROPÄISCHE PATENTANMELDUNG

(11) **EP 2 457 660 A1**
(43) Veröffentlichungstag der Anmeldung: **30.05.2012**
(21) Anmeldenummer: 12000777.8
(22) Anmeldetag: 25.03.2008
(51) Int. Cl.: B01L 3/00, G01N 33/558

(54) **Vorrichtung für einen Teststreifenhalter, Verfahren und Anordnung**

(30) Priorität: 24.03.2007 DE 102007014729
(62) Teilanmeldung aus: 08734397.6
(71) Anmelder: Matallana-Kielmann, Michael, 72116 Mössingen (DE)
(72) Erfinder: Matallana-Kielmann, Michael, 72116 Mössingen (DE)
(74) Vertreter: Castell, Klaus

(57) **Zusammenfassung**

Zur vereinfachten Verwendung eines Teststreifenhalters wird eine Vorrichtung mit einer Halte- oder Positioniereinrichtung vorgeschlagen, die eine Mischkammer, einen die Mischkammer verschließenden Deckel und eine öffenbare Fluidöffnung für einen Fluiddurchgang von der Mischkammer zum Teststreifenhalter aufweist.

Dies ermöglicht es, auf einfache Art und Weise den Teststreifenhalter in die Vorrichtung einzustecken und in der Mischkammer der Vorrichtung eine Probe vorzubereiten. Die Vorrichtung ist mit einer verschließbaren Mischkammer versehen, in der zur Verbesserung der Sensitivität des Teststreifens ein Reaktionspartner wie beispielsweise ein Goldkunjugat in der Probe aufgelöst werden kann.

## Beschreibung

Die Erfindung betrifft eine Vorrichtung für einen Teststreifenhalter, ein Verfahren zur Verwendung eines Teststreifenhalters und einer Anordnung aus einer derartigen Vorrichtung und einem in die Vorrichtung gesteckten Teststreifenhalter.

Teststreifenhalter sind beispielsweise für Lateral Flow Immunoassay Tests bekannt. Diese Teststreifenhalter enthalten einen Teststreifen, der an seinem einen Ende mit einer Probe in Verbindung gebracht wird und am anderen Ende mit einem Sammelpad in Verbindung steht, in dem durch den Teststreifen geflossenes Probematerial gesammelt wird. Der Teststreifen kann eine nitrocellulose Membran aufweisen, auf der Testlinien und Kontrolllinien angeordnet sind, die mit der Probe reagieren und auf denen eine Reaktion ablesbar ist.

Derartige Teststreifenhalter erleichtern den Umgang mit einem Teststreifen und das Aufgeben einer Probe auf den Teststreifen sowie das Ablesen des Ergebnisses.

Der Erfindung liegt die Aufgabe zu Grunde die Verwendung derartiger Teststreifenhalter noch weiter zu erleichtern.

Diese Aufgabe wird mit einer Vorrichtung für einen Teststreifenhalter gelöst, die eine Haltereinrichtung oder Positioniereinrichtung, eine Mischkammer, einen die Mischkammer verschließenden Deckel und eine öffenbare Fluidöffnung für einen Fluiddurchgang von der Mischkammer zum Teststreifenhalter aufweist.

Die mit einem Deckel verschließbare Mischkammer ermöglicht es, eine Probe vor der Beaufschlagung des Messstreifens mit einem weiteren Reaktionspartner zu mischen, gegebenenfalls reagieren zu lassen und zu inkubieren. Die öffenbare Fluidöffnung ermöglicht es, eine derart vorbereitete Probe auf einfache Art und Weise zum Messstreifen fließen zu lassen.

Während bisher die Proben für viele Tests aufwendig vorbehandelt werden mussten, erleichtert die Vorrichtung die Vorbereitung der Probe und das Aufbringen der Probe auf den Teststreifen. Die Halte- oder Positioniereinrichtung erlaubt es, die Mischkammer in der optimalen Position relativ zu einem Teststreifenhalter zu positionieren und vorzugsweise in dieser Position auch zu halten. Dadurch kann eine Probe sauber von der Mischkammer auf den Teststreifen überführt werden.

Die Vorrichtung ist derart einfach und kostengünstig herzustellen, dass sie als Kunststoffspritzteil ein Einmalprodukt darstellen kann, das nach der Verwendung mit dem Teststreifen und dem Teststreifenhalter entsorgt wird.

Vorteilhaft ist es, wenn die Halte- oder Positioniereinrichtung für ein Einschieben eines Teststreifenhalters ausgebildet ist. Die Einrichtung kann auch auf den Teststreifenhalter aufgepresst und beispielsweise mit ihm verrastet werden. Eine einfache Ausführungsvariante sieht hingegen vor, dass der Teststreifenhalter in die Vorrichtung eingeschoben wird und dadurch optimal relativ zur Mischkammer positioniert und gehalten wird.

Dies wird auf einfache Art und Weise dadurch erzielt, dass die Halte- oder Positioniereinrichtung zwei Wangen zur Positionierung eines Teststreifenhalters relativ zur Fluidöffnung aufweist. Diese zwei Wangen, die noch durch weitere Wangen und Anschläge ergänzt werden können, bilden eine Aufnahme für den Teststreifenhalter, um die Probe ohne Verschmutzungen sauber von der Mischkammer auf den Teststreifenhalter zu überführen.

Eine vorteilhafte Ausführungsvariante sieht vor, dass die Mischkammer einen in eine flüssige Phase überführbaren Reaktionspartner enthält. Dieser beispielsweise als fester Stoff in der Mischkammer vorliegende Reaktionspartner reagiert erst dann, wenn er mit einer flüssigen Probe in Verbindung gelangt und durch ein Hin- und Herbewegen der Probe in der Mischkammer gelöst wird. Der Reaktionspartner muss somit nicht erst abgemessen und in der richtigen Dosierung der Mischkammer zugegeben werden, da er sich bereits in der vorgefertigten Mischkammer befindet.

Um eine optimale Füllung der Mischkammer zu gewährleisten, wird vorgeschlagen, dass die Mischkammer eine Füllstandsmarkierung aufweist. Dies kann eine Farbmarkierung oder eine spezielle Struktur auf der Innenseite der Mischkammer sein, die dem Benutzer vorgibt, wie viel Probevolumen in die Mischkammer einzufüllen ist.

Eine besonders saubere Arbeitsweise konnte dadurch erzielt werden, dass die Mischkammer einen oberen Öffnungsbereich mit einer umlaufenden Vertiefung aufweist. Aus der Mischkammer austretende Materialien oder auch eine auf den oberen Rand auftreffende Probe sammelt sich dann in der umlaufenden Vertiefung, damit beim Schütteln der Vorrichtung eine Verschmutzung der Vorrichtung vermieden wird.

Alternativ oder kumulativ kann die Mischkammer an ihrem oberen Öffnungsbereich auch eine abgesenkte Ebene aufweisen, um Verschmutzungen zu vermeiden.

Ein einfacher Aufbau ergibt sich dadurch, dass der Deckel an der der Fluidöffnung gegenüber liegenden Seite der Mischkammer angeordnet ist. Beispielsweise kann der Deckel an der Oberseite der Mischkammer angeordnet sein, während die Fluidöffnung an der Unterseite der Mischkammer angeordnet ist.

Ein Verlieren des Deckels wird dadurch vermieden, dass der Deckel mit der Mischkammer verbunden ist. Vorzugsweise ist der Deckel mittels einer Lasche mit der Mischkammer verbunden. Diese Lasche ist flexibel und vorzugsweise elastisch. Eine derartige Lasche kann den Deckel relativ zur Mischkammer positionieren und dadurch das Aufsetzen des Deckels auf die Mischkammer erleichtern.

Eine gute Abdichtung des Deckels wird dadurch erzielt, dass der Deckel einen umlaufenden in die Mischkammer einführbaren Rand aufweist.

Weitere vorteilhafte Funktionen erschließen sich, wenn der Deckel zwei Schließpositionen aufweist. Vorteilhaft ist es, wenn in einer ersten Schließposition die Mischkammer abgedichtet wird und bei einer Bewegung des Deckels in eine zweite Schließposition die Fluidöffnung geöffnet wird.

Dies wird auf einfache Art und Weise dadurch erzielt, dass am Deckel ein Dorn angeordnet ist. Ein derartiger Dorn kann in der zweiten Schließposition die Fluidöffnung öffnen, indem er beispielsweise auf einen die Fluidöffnung abdeckenden Deckel drückt und die Fluidöffnung an einer Sollbruchstelle öffnet.

Um mehrere gleiche oder unterschiedliche Proben gleichzeitig behandeln zu können, wird vorgeschlagen, dass die Vorrichtung mehrere Mischkammern aufweist. Die in den Mischkammern vorbehandelten Proben können anschließend auf unterschiedliche Messstreifen aufgebracht werden. Hierzu kann entweder ein Teststreifenhalter mehrere Teststreifen aufweisen oder die Halte- und Positioniereinrichtung ist derart ausgebildet, dass sie mit mehreren Teststreifenhaltern zusammenwirken kann.

Insbesondere bei der Verwendung der Vorrichtung mit mehreren Teststreifen wird vorgeschlagen, dass sie eine Wandung aufweist, die bei geöffneter Fluidöffnung den Fluidfluss von der Mischkammer zum jeweiligen Teststreifen kanalysiert. Eine derartige Wandung kann beispielsweise durch einen umlaufenden Rand ausgebildet sein.

Der Umgang mit Teststreifenhaltern wird auch durch ein Verfahren zur Verwendung eines Teststreifenhalters erleichtert, bei dem ein Teststreifen in einem Teststreifenhalter angeordnet wird und der Teststreifenhalter an der zuvor beschriebenen Vorrichtung angeordnet wird. Vorzugsweise wird der Teststreifenhalter hierbei in die Vorrichtung eingeschoben.

Ein derartiges Verfahren ermöglicht es, eine Probe in die Mischkammer zu geben und zu schütten. Hierzu wird vorzugsweise in der Mischkammer ein geeigneter Reaktionspartner von der festen in die flüssige Phase überführt.

Vorteilhaft ist es, wenn die Probe in der Probenkammer eine Zeit inkubiert wird. Anschließend ist vorgesehen, dass mit einem Dorn im Deckel der Probenkammerboden durchstoßen wird. Dies ermöglicht es, dass die Probe aus der Vorrichtung auf einen Teststreifen im Teststreifenhalter fließt.

Auf dem Teststreifen ist vorzugsweise mindestens eine Linie vorhanden. Vorteilhaft ist es, wenn dort eine Test- und eine Kontrolllinie angeordnet sind. Eine derartige Linie kann in Form einer punktförmigen Fläche ausgebildet sein, alternativ kann auch eine viereckige Fläche oder eine Fläche in Form eine Pads vorgesehen sein.

Das Verfahren ermöglicht es, nach einer für den Test geeigneten Zeit ein Ergebnis abzulesen.

Ein derartiges Verfahren eignet sich vor allem für Teststreifenhalter die einen Lateral Flow Immunoassay Test aufweisen.

Die Erfindung betrifft letztlich auch eine Anordnung aus einer zuvor beschriebenen Vorrichtung und einem in die Vorrichtung gestreckten Teststreifenhalter, wobei dieser Teststreifenhalter insbesondere einen Lateral Flow Immunoassay Test enthält.

Im Folgenden wird die Erfindung an Hand eines Ausführungsbeispiels näher erläutert.

Es zeigt:
- Figur 1: eine schematische perspektivische Ansicht einer Vorrichtung mit einem Teststreifenhalter,
- Figur 2: einen Schnitt durch die in Figur 1 gezeigte Vorrichtung mit geöffnetem Deckel,
- Figur 3: einen Schnitt durch die in Figur 1 gezeigte Vorrichtung mit dem Deckel in einer ersten Schließposition und
- Figur 4: einen Schnitt durch die in Figur 1 gezeigte Vorrichtung mit einem Deckel in einer zweiten Schließposition.

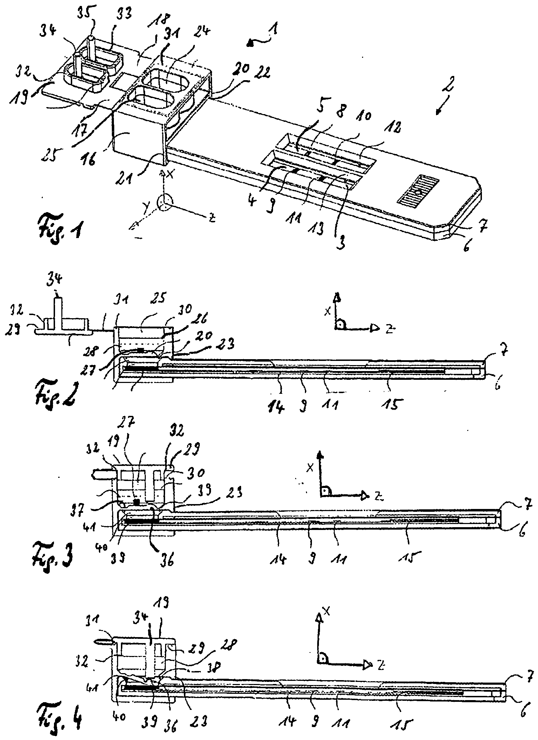

Die Figur 1 zeigt die Vorrichtung 1 für einen Teststreifenhalter 2, in dem ein Lateral Flow Immunoassay Test 3 mit zwei Teststreifen 4 und 5 angeordnet ist.

Der Teststreifenhalter 2 besteht aus zwei miteinander verbunden schalenförmigen Teilen 6, 7, zwischen denen die Teststreifen 4, 5 aufgenommen sind. Die Teststreifen 6, 7 haben jeweils zwei Testlinien, die als Testlinien 8, 9 und als Kontrolllinien 10, 11 ausgebildet sind. Das Ergebnis kann durch ein in dem oberen schalenförmigen Teil 7 ausgebildetes Reaktionsfenster 12, 13 abgelesen werden.

Im Teststreifenhalter 2 befindet sich eine Nitrocellulosemembran 14, die Probevolumen zu den Testlinien 8, 9 und den Kontrolllinien 10, 11 fließen lässt. Danach sammelt sich die Probe in einem Sammelpad 15 an einem Ende des Teststreifenhalters.

Die Vorrichtung 1 besteht aus einem Gehäuse 16, an dem über zwei flexible Laschen 17 und 18 ein Deckel 19 befestigt ist. Das Gehäuse 16 hat in seinem unteren Bereich ein Halte- und Positioniereinrichtung 20, die mit zwei Wangen 21, 22 den Teststreifenhalter 2 hält und positioniert. Der Teststreifenhalter 2 kann somit wie eine Schublade in das Gehäuse 16 eingeschoben werden. Dabei dient eine Hinterschneidung 23 für eine einrastende Befestigung des Teststreifenhalters 2 im Gehäuse 16.

Oberhalb der Halte- und Positioniereinrichtung 20 sind im Gehäuse zwei Mischkammern 24 und 25 vorgesehen, in die eine Probe bei geöffnetem Deckel 19 eingeführt werden kann. Die Probe wird dabei bis zur Markierung 26 in die Mischkammer gefüllt.

Dabei bedeckt die Probe einen Reaktionspartner 27, der in zunächst fester Phase am Boden der Mischkammer angeordnet und dort befestigt ist. Ein für einen Lateral Flow Immunoassay Test übliche Reaktionspartner ist beispielsweise ein Goldkunjugat, das in den Mischkammern 24 und 25 von der festen Phase in eine flüssige Phase gebracht werden kann, indem der Reaktionspartner in der Probe aufgelöst wird. Dadurch wird die Sensitivität des Immunoassay Tests verbessert.

Die in die Mischkammer 25 eingefüllte Probe 28 wird durch den in Figur 3 in aufgesetzter Position gezeigten Deckel 19 in der Mischkammer gehalten. Auch bei einem Schütteln der Vorrichtung 1 zum Auflösen des Reaktionspartners 27 gelangt somit keine Probe aus der Mischkammer.

Sollte sich beispielsweise beim Einfüllen am oberen Öffnungsbereich zwischen Probenkammerdeckelunterseite 29 und Probenkammeroberseite 30 Flüssigkeit sammeln, dient eine umlaufende Vertiefung 31 zur Aufnahme dieser Flüssigkeit, damit sie das Gehäuse 16 nicht verlässt. Anstelle einer umlaufenden Vertiefung 31 kann auch eine abgesenkte Ebene, die sich vorzugsweise zur Mischkammer hin öffnet, vorgesehen sein.

Der Deckel 19 weist zwei umlaufende in die Mischkammern 24 und 25 einführbare Ränder 32, 33 auf. Außerdem weist der Deckel 19 jeweils innerhalb der Ränder 32 und 33 einen Dorn 34, 35 auf.

Die Figur 3 zeigt wie der Deckel 19 in einer ersten Schließposition mit dem Rand 32 die Mischkammer abschließt, während zwischen der Probenkammerdeckelunterseite 29 und der Probenkammeroberkante 30 noch ein Abstand eingehalten wird. Der Dorn 34 steht in dieser Position oberhalb eines Anschlags 36, der am Mischkammerboden 37 vorgesehen ist.

Der Deckel 19 kann auch - wie in Figur 4 gezeigt - fester angedrückt werden, sodass die Ränder 32 und 33 weiter in die Mischkammern 24 und 25 eindringen. Dabei drücken die am Deckel angeordneten Dorne 34 und 35 auf den Kammerboden 37 und dort gegen den Anschlag 36. Hierdurch wird im Kammerboden eine Fluidöffnung 38 geöffnet, indem eine Sollbruchstelle 39 bricht und einen Fluiddurchgang von der Mischkammer 25 zum Teststreifenhalter 2 ermöglicht.

Nach Öffnen der Fluidöffnung 38 fließt die Probe 28 zunächst auf einen Probenpad 39 und von dort über die Nitrozellulosemembran 14 zu den Test- und Kontrollstreifen 9 und 11.

Damit insbesondere bei mehreren Fluidöffnungen 38 jede Probe auf den richtigen Teststreifen 4, 5 gelangt, ist eine Wandung 40, 41 vorgesehen, die bei geöffneter Fluidöffnung 38 den Fluidfluss von der Mischkammer 25 auf den jeweiligen Teststreifen 4 kanalisiert.

## Patentansprüche

1. Vorrichtung (1) für einen Teststreifenhalter (2) mit einer Halte- und/oder Positioniereinrichtung (20), einer Mischkammer (24, 25), einen die Mischkammer (24, 25) verschließenden Deckel (19) und einer öffenbaren Fluidöffnung (38) für einen Fluiddurchgang von der Mischkammer (24, 25) zum Teststreifenhalter (2), ***dadurch gekennzeichnet, dass*** der Deckel zwei Schließpositionen aufweist, wobei in einer ersten Schließposition die Mischkammer abgedichtet und bei einer Bewegung des Deckels in die zweite Schließposition die Fluidöffnung geöffnet wird.

2. Vorrichtung nach Anspruch 1, ***dadurch gekennzeichnet, dass*** die Halte- und/oder Positioniereinrichtung (20) für ein Einschieben eines Teststreifenhalters (2) ausgebildet ist.

3. Vorrichtung nach Anspruch 1 oder 2, ***dadurch gekennzeichnet, dass*** die Halte- und/oder Positioniereinrichtung (20) zwei Wangen (21, 22) zur Positionierung eines Teststreifenhalters (2) relativ zur Fluidöffnung (38) aufweist.

4. Vorrichtung nach einem der vorgehenden Ansprüche, ***dadurch gekennzeichnet, dass*** die Mischkammer (24, 25) einen in eine flüssige Phase überführbaren Reaktionspartner (27) enthält.

5. Vorrichtung nach einem der vorgehenden Ansprüche, ***dadurch gekennzeichnet, dass*** der Deckel (19) an der der Fluidöffnung (38) gegenüber liegenden Seite der Mischkammer (24, 25) angeordnet ist.

6. Vorrichtung nach einem der vorher gehenden Ansprüche, ***dadurch gekennzeichnet, dass*** die Lasche (17, 18) den Deckel (19) zur Mischkammer (24, 25) positioniert.

7. Vorrichtung nach einem der vorgehenden Ansprüche, ***dadurch gekennzeichnet, dass*** der Deckel (19) einen umlaufenden in die Mischkammer (24, 25) einführbaren Rand (32, 33) aufweist.

8. Vorrichtung nach einem der vorgehenden Ansprüche, ***dadurch gekennzeichnet, dass*** sie mehrere Mischkammern (24, 25) aufweist.

9. Vorrichtung nach einem der vorgehenden Ansprüche, ***dadurch gekennzeichnet, dass*** eine Wandung (40, 41) bei geöffneter Fluidöffnung (28) den Fluidfluss von der Mischkammer (25) zum jeweiligen Teststreifen (4, 5) kanalisiert.

10. Verfahren zur Verwendung eines Teststreifenhalters (2), bei dem ein Teststreifenhalter an der Vorrichtung nach einem der vorhergehenden Ansprüche angeordnet ist.

11. Verfahren nach Anspruch 10, ***dadurch gekennzeichnet, dass*** eine Probe in die Mischkammer (25) gegeben und geschüttelt wird.

12. Verfahren nach Anspruch 10 oder 11, ***dadurch gekennzeichnet, dass*** in der Mischkammer (24) ein geeigneter Reaktionspartner (27) von der festen in die flüssige Phase überführt wird.

13. Verfahren nach einem der Ansprüche 10 bis 12, ***dadurch gekennzeichnet, dass*** eine Probe in der Mischkammer (24) inkubiert wird.

14. Verfahren nach einem der Ansprüche 10 bis 13, ***dadurch gekennzeichnet, dass*** der Teststreifenhalter einen Lateral Flow Immunoassay Test aufweist.

15. Anordnung aus einer Vorrichtung nach einem der Ansprüche 1 bis 9 und einem in die Vorrichtung gesteckten Teststreifenhalter (2).
